# FASCICULE DE BREVET EUROPEEN

(11) **EP 2 032 200 B1**
(45) Date de publication et mention de la délivrance du brevet: **05.05.2021**
(21) Numéro de dépôt: 07729979.0
(22) Date de dépôt: 07.06.2007
(51) Int. Cl.: A61M 39/06, A61B 17/34, A61M 25/00

(54) **ADAPTATEUR POUR VALVE D'INTRODUCTEUR DANS LE CORPS HUMAIN OU ANIMAL**
ADAPTER FÜR EIN EINFÜHRUNGSVENTIL IN EINEM MENSCHLICHEN ODER TIERISCHEN KÖRPER
ADAPTOR FOR AN INTRODUCER VALVE IN A HUMAN OR ANIMAL BODY

(30) Priorité: 13.06.2006 FR 0652112; 25.10.2006 US 854090 P
(43) Date de publication de la demande: 11.03.2009
(73) Titulaire: Mialhe, Claude, 83300 Draguignan (FR)
(72) Inventeur: Mialhe, Claude, 83300 Draguignan (FR)
(74) Mandataire: Decobert, Jean-Pascal
(86) Numéro de dépôt international: PCT/EP2007/055615
(87) Numéro de publication internationale: WO 2007/144305

(56) Documents cités:
- EP-A- 0 834 279
- WO-A-00/32263
- WO-A2-00/74760
- FR-A1- 2 878 750
- US-A1- 2002 111 585

## Description

L'invention concerne un introducteur à valve pour le corps humain ou animal muni d'un adaptateur.

Elle trouvera en particulier son application dans le domaine de la chirurgie vasculaire.

Les instruments d'introduction dans le corps humain ou animal nécessitent la présence d'éléments obturateurs aptes à assurer l'étanchéité de l'introducteur.

Ceci est particulièrement vrai lors d'interventions endovasculaires avec introduction d'instruments chirurgicaux dans un introducteur où il faut éviter la fuite rétrograde de flux sanguins au travers de l'instrument introducteur.

On connaît déjà des valves pour instrument chirurgical déformables en torsion dans le but d'augmenter l'étanchéité de l'instrument.

Cette solution n'est pas pleinement satisfaisante car lors de l'introduction de plusieurs instruments chirurgicaux tels que des cathéters et des guides, il reste malgré cette valve un jour entre les instruments provoquant la fuite de flux sanguin.

Du document WO00/32263 on connaît un dispositif d'introduction dans le corps humain comprenant un corps principal allongé muni à son extrémité proximale d'une pluralité d'ouvertures destinées à recevoir des outils chirurgicaux. Le dispositif présente des problèmes d'étanchéité du fait que le corps principal allongé est directement introduit dans le corps humain et les outils au travers de celui-ci.

De plus ce système n'est pas très simple à manipuler puisque une fois le dispositif introduit aucun changement dans le diamètre ou le nombre des ouvertures à l'extrémité proximale ne peut être réalisé sans retirer l'ensemble du dispositif et donc entraîner la fuite de fluide du corps humain.

Il existe donc un besoin de proposer un dispositif permettant l'utilisation simultanée de plusieurs instruments chirurgicaux ou d'instruments de tailles fort différentes au travers d'un introducteur dans le corps humain ou animal avec une bonne étanchéité et une facilité de manipulation.

A cet effet, l'invention propose un introducteur à valve pour le corps humain ou animal comprenant un adaptateur dont la surface extérieure est apte à coopérer avec la valve, un volume intérieur avec au moins un conduit destiné à permettre le passage d'instruments chirurgicaux ou médicaux de corps allongé et des moyens d'étanchéité.

Selon un mode de réalisation, les conduits de l'adaptateur comprennent des moyens d'étanchéité à leur extrémité proximale et/ou à leur extrémité distale.

Avantageusement, l'adaptateur est composé d'un corps allongé de section cylindrique apte à coopérer avec une valve déformable en torsion.

Les moyens d'étanchéité des conduits ainsi que le corps allongé permettent une bonne étanchéité de l'adaptateur et de l'ensemble adaptateur - introducteur.

Avantageusement, les moyens d'étanchéité de l'extrémité distale sont aussi destinés à éviter une trop grande mobilité des cathéters, en les maintenant dans un certain axe.

Les moyens d'étanchéité de l'extrémité distale ont aussi un rôle de centrage.

Avantageusement, l'adaptateur comprend une surface externe apte à coopérer avec l'introducteur ainsi que des moyens d'attache à l'introducteur.

Avantageusement, l'adaptateur comprend un embout distal de forme conique destiné à améliorer l'insertion de l'adaptateur dans l'introducteur avantageusement en coopérant avec une valve plane de centrage de l'introducteur et/ou en favorisant l'ouverture de la valve d'étanchéité de l'introducteur, en particulier si l'introducteur est muni d'une valve d'étanchéité déformable en torsion.

Selon un mode de réalisation les conduits de l'adaptateur se composent d'une partie sensiblement longitudinale par rapport au corps de l'adaptateur et d'une partie proximale divergente. Cette disposition a l'avantage de faciliter la manipulation des instruments chirurgicaux introduits dans l'adaptateur.

Avantageusement, les centres des orifices proximaux des conduits se situent selon un cercle prédéterminé.

Selon un mode de réalisation, un canal se situe au centre du cercle prédéterminé par les orifices proximaux des conduits et avantageusement, ce canal possède les mêmes moyens d'étanchéité que les conduits.

Avantageusement ledit canal est rectiligne, afin de faciliter la manipulation des instruments chirurgicaux et en particulier le passage d'un fil de guidage dit guide wire.

D'autres buts et avantages apparaîtront au cours de la description qui suit d'un mode préféré de réalisation de l'invention qui n'en est cependant pas limitatif.

Il convient de rappeler que l'invention concerne un introducteur à valve pour le corps humain ou animal caractérisé par le fait que la valve est déformable en torsion et apte à coopérer avec un adaptateur par sa surface extérieure; et que le volume intérieur dudit adaptateur comprend au moins un conduit destiné à permettre le passage d'instruments chirurgicaux ou médicaux de corps allongé ; et que le conduit comprend des moyens d'étanchéité.

Suivant des variantes préférées de l'invention, l'introducteur est tel que :
- le conduit de l'adaptateur comprend des moyens d'étanchéité à son extrémité proximale,
- les moyens d'étanchéité à l'extrémité proximale du conduit de l'adaptateur comprennent une valve hémostatique,
- le conduit de l'adaptateur comprend des moyens d'étanchéité à son extrémité distale,
- les moyens d'étanchéité à l'extrémité distale du conduit de l'adaptateur comprennent une valve hémostatique,
- la valve hémostatique est maintenue entre un support et une rondelle,
- les centres des orifices proximaux des conduits de l'adaptateur se situent selon un cercle prédéterminé,
- un canal se situe au centre du cercle prédéterminé par les orifices proximaux des conduits de l'adaptateur et ledit canal est rectiligne.
- les conduits de l'adaptateur se composent d'une partie sensiblement longitudinale par rapport au corps allongé de l'adaptateur et d'une partie proximale divergente,
- les conduits de l'adaptateur ont des diamètres différents.
- l'adaptateur comporte un embout distal de forme conique,
- l'adaptateur comprend des moyens d'attache à l'introducteur,
- l'adaptateur comprend un corps allongé de section cylindrique.

Suivant des variantes préférées de l'invention, l'introducteur est tel
- qu'il comporte une valve déformable en torsion apte à coopérer avec un adaptateur par sa surface extérieure comme précédemment décrit,
- qu'il comporte une valve plane de centrage apte à coopérer avec l'extrémité distale de l'adaptateur de sorte à faciliter son insertion en la centrant.

Les dessins ci-joints sont donnés à titre d'exemples et ne sont pas limitatifs de l'invention. Ils représentent seulement un mode de réalisation de l'invention et permettront de la comprendre aisément.

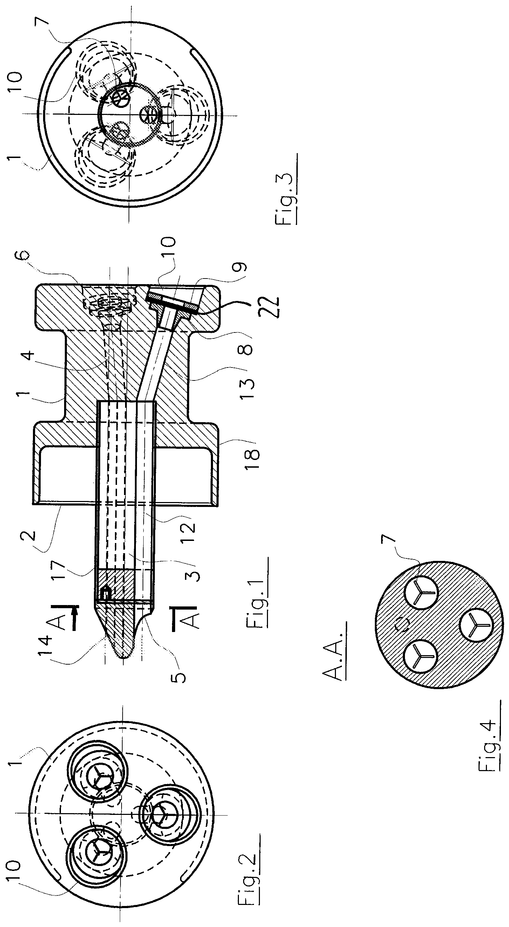

Figure 1 : vue en coupe longitudinale de l'adaptateur.
Figure 2 : vue en coupe à partir de l'extrémité distale de l'adaptateur selon la figure 1.
Figure 3 : vue en coupe à partir de l'extrémité proximale de l'adaptateur selon la figure 1.
Figure 4 : vue du corps allongé de l'adaptateur selon la coupe AA de la figure 1.

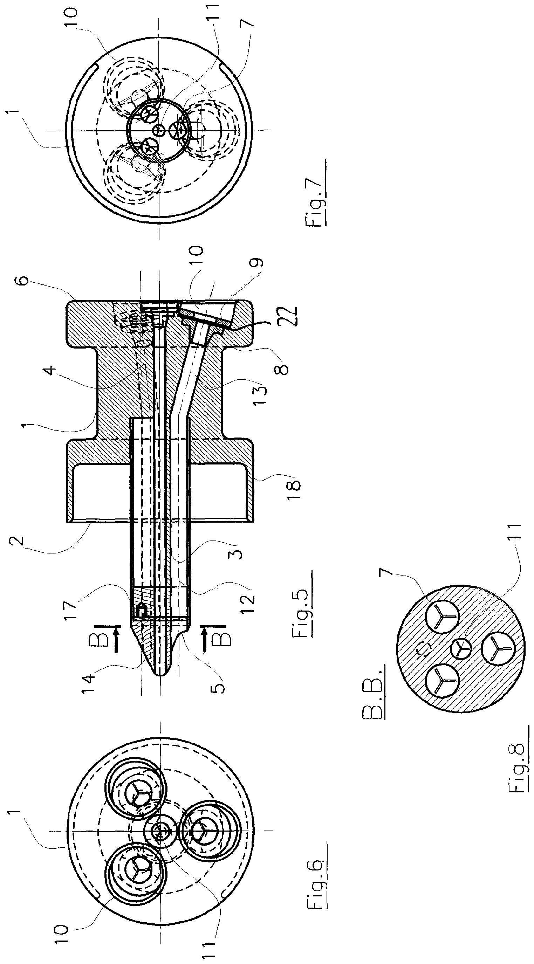

Figure 5 : vue en coupe longitudinale de l'adaptateur selon le mode de réalisation comprenant un canal central.
Figure 6 : vue en coupe à partir de l'extrémité distale de l'adaptateur selon la figure 5.
Figure 7 : vue en coupe à partir de l'extrémité proximale de l'adaptateur selon la figure 5.
Figure 8 : vue du corps allongé de l'adaptateur selon la coupe BB de la figure 5.

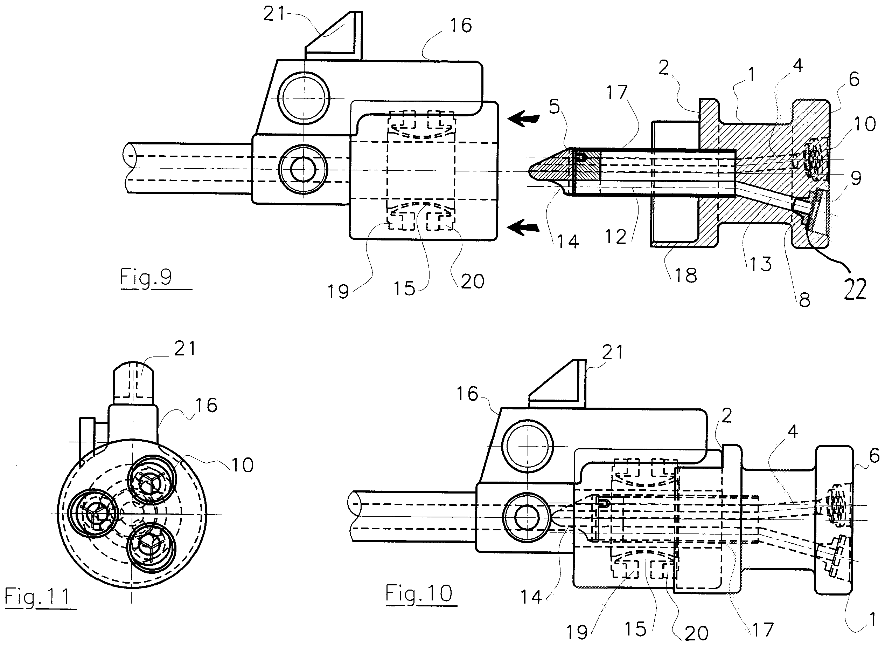

Figure 9 : vue en coupe longitudinale de l'adaptateur et de l'introducteur.
Figure 10 : vue en coupe longitudinale de l'adaptateur fixé sur l'introducteur.
Figure 11 : vue en coupe à partir de l'extrémité distale selon la figure 10.

L'adaptateur 1 pour valve 15 d'introducteur 16 dans le corps humain ou animal comprend une surface extérieure 2 apte à coopérer avec la valve 15.

L'adaptateur 1 comprend un corps allongé 17 avantageusement de section cylindrique apte à coopérer avec une valve 15 d'introducteur 16.

Avantageusement une sorte de jupe 18 vient partiellement recouvrir le corps allongé 17 de l'adaptateur 1.

Selon un mode de réalisation, la valve 15 de l'introducteur 16 est une valve déformable en torsion.

La valve 15 est formée d'une portion souple de forme sensiblement cylindrique, creuse en matériau étanche et flexible tel du silicone.

Les deux extrémités 19 et 20 de la valve 15 sont solidaires d'autres pièces servant à la torsion de la valve 15.

Si la portion souple n'est pas sollicitée alors son centre est complètement ouvert permettant le passage des instruments chirurgicaux ou médicaux au travers de la valve 15.

Au moyen d'un curseur 21, l'opérateur met en rotation l'extrémité 20 par l'intermédiaire d'un câble. Une torsion de la portion souple tend alors à créer une zone de striction sensiblement au milieu de la longueur de la portion souple.

Lorsqu'il souhaite introduire un objet au travers de la valve 15, l'opérateur actionne le curseur 21.

Il n'est pas exclu de mettre en rotation l'extrémité 19 au lieu de l'extrémité 20, ou de réaliser une rotation simultanée et opposée des deux extrémités 19 et 20.

Selon un mode de réalisation, l'introducteur comprend une première valve plane de centrage en amont de la valve 15 déformable en torsion.

Avantageusement, la valve plane de centrage est du type tricuspide ou punctiforme.

L'adaptateur 1 comprend dans son volume intérieur 3 au moins un conduit 4 destiné à permettre le passage d'instruments chirurgicaux ou médicaux de corps allongé.

Selon un mode de réalisation, l'adaptateur 1 comprend trois conduits 4.

Avantageusement, les conduits 4 comprennent des moyens d'étanchéité à leur extrémité proximale 6 et/ou distale 5.

Selon un mode de réalisation, les conduits 4 comprennent une valve hémostatique 22 préférentiellement du type tricuspide en leur extrémité proximale 6 et/ou une valve hémostatique 7 préférentiellement du type tricuspide en leur extrémité distale 5. On entend par tricuspide, une valve sous forme d'une rondelle en matériau souple du type silicone ou polymère au centre de laquelle trois entailles sont formées pour le passage d'instruments sous étanchéité.

Avantageusement, la valve hémostatique 22 est maintenue entre un support 8 et une rondelle 9. Avantageusement, le support 8 et la rondelle 9 sont percés d'un passage de même diamètre que les conduits 4 destinés à permettre l'introduction des instruments chirurgicaux.

Selon un mode de réalisation, les conduits 4 comprennent un bouchon amovible (non représenté) en leur extrémité proximale 6 destiné à boucher les conduits 4 non utilisés, en se plaçant par-dessus la valve hémostatique 22 et sa rondelle 9..

Selon un mode de réalisation, les conduits 4 se composent d'une partie longitudinale 12 par rapport au corps allongé 17 de l'adaptateur 1 et d'une partie proximale divergente 13. Cette configuration facilite la manipulation des instruments chirurgicaux introduits dans l'adaptateur.

Les centres des orifices proximaux 10 des conduits 4 se situent avantageusement selon un cercle prédéterminé et selon un mode de réalisation un canal 11 se situe au centre du cercle prédéterminé par les orifices proximaux 10 des conduits 4.

Avantageusement ledit canal 11 possède les mêmes moyens d'étanchéité que les conduits 4. Selon un mode de réalisation, le canal 11 est rectiligne afin de faciliter la manipulation des instruments chirurgicaux et en particulier le passage d'un fil de guidage dit guide wire.

Selon un mode de réalisation, l'adaptateur 1 comprend un embout distal 14 de forme conique destiné à guider et à centrer l'insertion de l'adaptateur 1 dans l'introducteur 16. L'embout distal 14 appuie en se centrant sur la première valve plane située en amont de la valve 15 déformable en torsion qui a été préalablement ouverte par l'opérateur. L'embout distal 14 permet une étanchéité continue entre l'adaptateur 1 et l'introducteur 16, en appuyant de manière continue sur la première valve en amont puis, la valve 15 déformable en torsion vient appuyer sur le corps allongé 17 de l'adaptateur 1.

Selon un autre mode de réalisation, l'adaptateur 1 comprend un embout distal 14 de forme conique destiné à faciliter l'insertion de l'adaptateur 1 dans l'introducteur 16 en ouvrant la valve 15, en particulier si la valve 15 est déformable en torsion. C'est le cas d'une valve déformable en torsion en position fermée au départ. L'embout distal 14 exerce une pression tendant à ouvrir progressivement la valve qui continue cependant à s'appliquer sur l'adaptateur, sans rupture d'étanchéité.

Selon un mode de réalisation, les conduits 4 ont des diamètres différents destinés à recevoir divers types d'instruments chirurgicaux.

### REFERENCES

1. Adaptateur
2. Surface extérieure
3. Volume intérieur
4. Conduit
5. Extrémité distale
6. Extrémité proximale
7. Valve hémostatique
8. Support valve
9. Rondelle
10. Orifice proximal
11. Canal
12. Partie longitudinale
13. Partie proximale divergente
14. Embout distal
15. Valve
16. Introducteur
17. Corps allongé
18. Jupe
19. Extrémité fixe de la valve
20. Extrémité mobile de la valve
21. Curseur
22. Valve hémostatique

## Revendications

1. Introducteur (16) à valve (15) pour le corps humain ou animal **caractérisé par le fait**
**qu'**il comporte un adaptateur (1) et que la valve (15) est configurée pour se déformer en torsion et reçoit en son centre l'adaptateur (1) de sorte à s'appuyer sur une surface extérieure (2) dudit adaptateur (1) ; et
**que** le volume intérieur (3) dudit adaptateur (1) comprend au moins un conduit (4) destiné à permettre le passage d'instruments chirurgicaux ou médicaux de corps allongé ; et
**que** le conduit (4) comprend des moyens d'étanchéité à son extrémité proximale (6).

2. Introducteur (16) à valve (15) selon la revendication 1 dans lequel les moyens d'étanchéité à l'extrémité proximale du conduit (4) comprennent une valve hémostatique (22).

3. Introducteur (16) à valve (15) selon une quelconque des revendications 1 à 2 **caractérisé par le fait**
**que** le conduit (4) comprend des moyens d'étanchéité à son extrémité distale (5).

4. Introducteur (16) à valve (15) selon la revendication 3 **caractérisé par le fait**
**que** les moyens d'étanchéité à l'extrémité distale (5) du conduit (4) comprennent une valve hémostatique (7).

5. Introducteur (16) à valve (15) selon une quelconque des revendications précédentes **caractérisé par le fait**
**que** la valve hémostatique (22) est maintenue entre un support (8) et une rondelle (9).

6. Introducteur (16) à valve (15) selon une quelconque des revendications 1 à 5 **caractérisé par le fait**
**que** les centres des orifices proximaux (10) des conduits (4) se situent selon un cercle prédéterminé.

7. Introducteur (16) à valve (15) selon la revendication 6 **caractérisé par le fait**
**qu'**un canal (11) se situe au centre du cercle prédéterminé par les orifices proximaux (10) des conduits (4) et que ledit canal est rectiligne.

8. Introducteur (16) à valve (15) selon une quelconque des revendications 1 à 7 **caractérisé par le fait**
**que** les conduits (4) se composent d'une partie sensiblement longitudinale (12) par rapport au corps allongé (17) de l'adaptateur (1) et d'une partie proximale divergente (13).

9. Introducteur (16) à valve (15) selon une quelconque des revendications 1 à 8 **caractérisé par le fait**
**que** les conduits (4) ont des diamètres différents.

10. Introducteur (16) à valve (15) selon une quelconque des revendications 1 à 9 **caractérisé par le fait**
**que** l'adaptateur comporte un embout distal (14) de forme conique.

11. Introducteur (16) à valve (15) selon une quelconque des revendications 1 à 10 **caractérisé par le fait**
**que** l'adaptateur comprend des moyens d'attache à l'introducteur (16).

12. Introducteur (16) à valve (15) selon les revendications 1 à 11 **caractérisé par le fait**
**que** l'adaptateur comprend un corps allongé (17) de section cylindrique sur lequel s'appuie la valve 15.

13. Introducteur (16) à valve (15) selon une quelconque des revendications précédentes **caractérisé par le fait**
**qu'**il comporte une valve plane de centrage coopérant avec l'extrémité distale de l'adaptateur (1) de sorte à faciliter son insertion en la centrant.

## Patentansprüche

1. Einführer (16) mit Ventil (15) für den menschlichen oder tierischen Körper, **dadurch gekennzeichnet,**
**dass** er einen Adapter (1) aufweist und dass das Ventil (15) ausgelegt ist, um sich durch Torsion zu verformen und in seinem Zentrum den Adapter (1) derart aufnimmt, dass eine Abstützung auf einer Außenfläche (2) des Adapters (1) erfolgt; und
**dass** das Innenvolumen (3) des Adapters (1) mindestens eine Führung (4) umfasst, die bestimmt ist, den Durchgang chirurgischer oder medizinischer Instrumente mit länglichem Körper zu erlauben; und
**dass** die Führung (4) Dichtungsmittel an ihrem proximalen Ende (6) umfasst.

2. Einführer (16) mit Ventil (15) nach Anspruch 1, wobei die Dichtungsmittel am proximalen Ende der Leitung (4) ein hämostatisches Ventil (22) umfassen.

3. Einführer (16) mit Ventil (15) nach einem der Ansprüche 1 bis 2, **dadurch gekennzeichnet,**
**dass** die Führung (4) Dichtungsmittel an ihrem distalen Ende (5) umfasst.

4. Einführer (16) mit Ventil (15) nach Anspruch 3, **dadurch gekennzeichnet,**
**dass** die Dichtungsmittel am distalen Ende (5) der Führung (4) ein hämostatisches Ventil (7) umfassen.

5. Einführer (16) mit Ventil (15) nach einem der vorangehenden Ansprüche, **dadurch gekennzeichnet,**
**dass** das hämostatische Ventil (22) zwischen einem Halter (8) und einer Scheibe (9) gehalten wird.

6. Einführer (16) mit Ventil (15) nach einem der Ansprüche 1 bis 5, **dadurch gekennzeichnet,**
**dass** sich die Zentren der proximalen Öffnungen (10) der Führungen (4) gemäß einem vorher festgelegten Kreis befinden.

7. Einführer (16) mit Ventil (15) nach Anspruch 6, **dadurch gekennzeichnet,**
**dass** sich ein Kanal (11) im Zentrum des durch die proximalen Öffnungen (10) der Führungen (4) vorher festgelegten Kreises befindet und dass der Kanal gerade ist.

8. Einführer (16) mit Ventil (15) nach einem der Ansprüche 1 bis 7, **dadurch gekennzeichnet,**
**dass** sich die Führungen (4) aus einem in Bezug auf den länglichen Körper (17) des Adapters (1) etwa länglichen Teil (12) und einem divergierenden proximalen Teil (13) zusammensetzen.

9. Einführer (16) mit Ventil (15) nach einem der Ansprüche 1 bis 8, **dadurch gekennzeichnet,**
**dass** die Führungen (4) unterschiedliche Durchmesser haben.

10. Einführer (16) mit Ventil (15) nach einem der Ansprüche 1 bis 9, **dadurch gekennzeichnet,**
**dass** der Adapter eine distale Spitze (14) konischer Form aufweist.

11. Einführer (16) mit Ventil (15) nach einem der Ansprüche 1 bis 10, **dadurch gekennzeichnet,**
**dass** der Adapter Befestigungsmittel am Einführer (16) umfasst.

12. Einführer (16) mit Ventil (15) nach den Ansprüchen 1 bis 11, **dadurch gekennzeichnet,**
**dass** der Adapter einen länglichen Körper (17) mit zylindrischem Querschnitt umfasst, auf dem sich das Ventil (15) abstützt.

13. Einführer (16) mit Ventil (15) nach einem der vorangehenden Ansprüche, **dadurch gekennzeichnet,**
**dass** er ein ebenes Zentrierventil aufweist, das mit dem distalen Ende des Adapters (1) derart zusammenwirkt, dass sein Einsetzen durch Zentrieren erleichtert wird.

## Claims

1. Valve (15) introducer (16) for the human or animal body **characterised in that** it includes an adaptor (1) and the valve (15) is configured to be torsionally deformed and receives at the centre thereof the adaptor (1) so as to bear on an outer surface (2) of said adaptor (1); and the inner volume (3) of said adaptor (1) comprises at least one conduit (4) intended to allow surgical or medical instruments having an elongate body to pass through; and the conduit (4) comprises sealing means at the proximal end (6) thereof.

2. Valve (15) introducer (16) according to claim 1 wherein the sealing means at the proximal end of the conduit (4) comprise a haemostatic valve (22).

3. Valve (15) introducer (16) according to any one of claims 1 to 2 **characterised in that** the conduit (4) comprises sealing means at the distal end (5) thereof.

4. Valve (15) introducer (16) according to claim 3 **characterised in that** the sealing means at the distal end (5) of the conduit (4) comprise a haemostatic valve (7).

5. Valve (15) introducer (16) according to any one of the preceding claims **characterised in that** the haemostatic valve (22) is held between a support (8) and a washer (9).

6. Valve (15) introducer (16) according to any one of claims 1 to 5 **characterised in that** the centres of the proximal orifices (10) of the conduits (4) are located along a predetermined circle.

7. Valve (15) introducer (16) according to claim 6 **characterised in that** a channel (11) is located at the centre of the circle predetermined by the proximal orifices (10) of the conduits (4) and said channel is rectilinear.

8. Valve (15) introducer (16) according to any one of claims 1 to 7 **characterised in that** the conduits (4) are composed of a substantially longitudinal portion (12) with respect to the elongate body (17) of the adaptor (1) and of a divergent proximal portion (13).

9. Valve (15) introducer (16) according to any one of claims 1 to 8 **characterised in that** the conduits (4) have different diameters.

10. Valve (15) introducer (16) according to any one of claims 1 to 9 **characterised in that** the adaptor includes a distal end piece (14) of conical shape.

11. Valve (15) introducer (16) according to any one of claims 1 to 10 **characterised in that** the adaptor comprises attachment means to the introducer (16).

12. Valve (15) introducer (16) according to claims 1 to 11 **characterised in that** the adaptor comprises an elongate body (17) of cylindrical cross-section whereon the valve 15 bears.

13. Valve (15) introducer (16) according to any one of the preceding claims **characterised in that** it includes a planar centring valve cooperating with the distal end of the adaptor (1) so as to facilitate the insertion thereof by centring it.
